(19) Europäisches Patentamt

European Patent Office

Office européen des brevets

(11) Publication number: **0 275 215 B1**

# (12) EUROPEAN PATENT SPECIFICATION

(45) Date of publication of patent specification: **08.04.92**  (51) Int. Cl.5: **A61K 49/00**

(21) Application number: **88300344.4**

(22) Date of filing: **15.01.88**

(54) Contrast agents for the gastrointestinal tract.

(30) Priority: **16.01.87 GB 8701054**

(43) Date of publication of application:
**20.07.88 Bulletin 88/29**

(45) Publication of the grant of the patent:
**08.04.92 Bulletin 92/15**

(84) Designated Contracting States:
**AT BE CH DE FR GB IT LI LU NL SE**

(56) References cited:
**EP-A- 0 210 043**
**GB-A- 2 137 612**

(73) Proprietor: **GUERBET S.A.**
**15, rue des Vanesses**
**F-93430 Villepinte(FR)**

(72) Inventor: **Sadler, Peter John**
**40 Albion Road Pitstone**
**Leighton Buzzard Bedfordshire LU7 9AY(GB)**
Inventor: **McEwen, Andrew Bruce**
**4 Nightingale Court 54 Thrale Road**
**Streatham SW16 1NY(GB)**

(74) Representative: **Polus, Camille et al**
**c/o Cabinet Lavoix 2, Place d'Estienne**
**d'Orves**
**F-75441 Paris Cedex 09(FR)**

## Description

This invention relates to a contrast agent for investigating the gastrointestinal (GI) tract by nuclear magnetic resonance (NMR) scanning. Contrast agents are used in NMR scanning to alter the T1 and T2 relaxation times of protons in their vicinity. Analysis of proton relaxation times is used to determine where in the body, or other object being scanned, the contrast agent has become located and this in turn can provide information about the internal structure of the body. Due to similar relaxation behaviour of the GI tract and surrounding tissues, discrimination is not possible in the absence of contrast agents. The motility of various regions of the gut also requires the use of faster pulse sequences to avoid movement artefacts. If a contrast agent could be administered orally or rectally and thereby cause an alteration in the T1 or T2 relaxation times of the adjacent protons, it would be possible to generate an NMR scanning image that might give valuable information about the state of the GI tract.

Present methods for studying the GI tract involve the use of radioopaque media and an external source of X-radiation. These high density contrast media fill the GI tract and prevent the passage of X-rays through the soft tissue so that the organ is seen in contrast with surrounding tissues. This technique sees only the organ of interest and gives no information on surrounding tissues. If these media are used in the study of intestinal transit times, gastric emptying times or in monitoring the passage of material through the GI tract, the patient can be subjected to undesirable high levels of high energy radiation.

The most commonly used radioopaque agent in GI imaging is barium sulphate. The high density of this compound often results in intestinal compaction, thereby complicating disease states in which intestinal obstructions are already present. The high density of this compound also results in long transit times which increase the time of exposure to X-radiation. Administration of barium sulphate has resulted in intra- and extraperitoneal intestinal perforation and isolated cases of vascular invasion.

In an attempt to avoid these problems water soluble reagents e.g. Hypaque® were developed as contrast media. These are iodinated compounds which relay on the radiodensity of iodine to provide contrast in the presence of X-radiation. Some of these compounds tend to be hyperosmotic and result in large volumes of water diffusing into the GI system. The resultant water imbalance can lead to diarrhoea and dehydration and the use of these compounds is contraindicated where these conditions would present the patient with problems.

Newer generations of radiopharmaceuticals use internal sources of radiation from internalised complexes of 99m-technetium, 113m-indium or 111-indium. These radionucleides have suitably short half lives for use in human investigations, coupled with low energy $\gamma$-radiation. However even though the radiation doses are low it would be desirable to use non-radioactive contrast media, especially where the investigations may extend as a series over long periods of time, or where due to conditions such as intestinal blockage the residence time is likely to be long.

Non-radiological techniques used to date have included the use of inert compounds such as carbon black or chromic oxide to determine the time between oral dosing and appearance of the compound in the faeces. No information is available as to its route or any problems, other than low motility, with the GI tract and no visualisation is possible.

It can be seen that diagnosis of GI tract conditions would be greatly aided by an effective GI visualising agent free from the problems described above.

The present invention provides a paramagnetic material of controllable particle size which is easily distributed through the gastric contents. The particulate will pass through the GI tract unchanged and can be monitored using NMR relaxation times to visualise the required organ. In this invention suitable paramagnetic metal ions including those of the lanthanide elements with atomic numbers 58 to 70, and those of the transition metals with atomic numbers 21 to 29, 42 and 44 are bound to an insoluble carrier, either in association with a suitable complexant or by precipitation of an insoluble salt. By this means physiologically insoluble compounds may be prepared to produce an effective and safe GI scanning agent.

In EP-A-0 210 043 we have shown that metal complexes with phosphonate ligands show an affinity for hydroxyapatite crystals and are thus liable to locate in vivo in regions of bone metabolism. It was shown that these metal phosphonate-hydroxyapatite adducts retained the ability to affect proton relaxation times in vitro and their potential use as diagnostic aids in their own right was claimed. The present invention extends this application to show how other insoluble particulates can also act as physiologically acceptable carriers for use in NMR scanning.

The invention concerns a contrast agent for investigating GI tract function and disorders by NMR scanning which agent comprises a complex formed between a) an amino di- or poly-phosphonate in which the phosphonate groups comprise different carbon atoms, and b) a paramagnetic metal ion, in substantially permanent association with an insoluble particulate carrier. The invention also concerns an agent compris-

2

ing a paramagnetic metal ion insolubilised on the surface of an insoluble particulate by chemical methods such as precipitation using phosphate or hydroxide ions.

Preferred di- or poly-phosphonates contain the groups $-N(CH_2PO_3H_2)_2$ at one or more, generally one or two, positions in the molecule. These phosphonates may have the formula:-

$$YN(CH_2PO_3H_2)_2$$

or

$$Z[N(CH_2PO_3H_2)_2]_2$$

where each of Y and Z may be an aliphatic or aromatic groups containing 1 to 12 carbon atoms which may also contain one or more hydroxyl, ether, carboxylate, amine, sulphonate, or aminomethylphosphonate groups or non toxic salts thereof.

A polyphosphonate may for example have the general formula:-

$$NX_2.CH_2.PO_3.H_2$$

where the two groups X are the same or different and each is $-CH_2PO_3H_2$ or $-(CH_2)nN(CH_2PO_3H_2)_2$, and n is up to 10 preferably 2 or 6, or a non toxic salt thereof.

Examples of specific polyphosphonates are nitrilotrismethylenephosphonate (NTMP) , hexamethylenediaminetetramethylenephosphonate (HDTMP) , ethylenediaminetetramethylenephosphonate (EDTMP) , diethylenetriaminepentamethylenephosphonate (DPTMP) , metaxylenediaminetetramethylenephosphonate (MXDTMP) , and non toxic salts thereof.

Suitable paramagnetic metal ions are well known in the literature and include those of the lanthanide elements with atomic numbers 58 to 70 and those of the transition metals with atomic numbers 21 to 29, 42 and 44. Preferred are Mn(II), Cu(II), Fe(II), Gd(III), Fe(III), Cr(III), Dy(III), and V(IV). Factors affecting the choice of metal ion are its paramagnetic properties, the stability and solubility of the metal ion phosphonate complex, the extent to which the metal complex binds to the carrier and its subsequent stability, its toxicity, and the extent to which the metal ion in the adduct interacts with water so as to affect proton relaxation times.

Suitable carriers include hydroxyapatite, whose ability to bind metal phosphonate groups has been well documented. In general the pharmaceutically-acceptable, substantially physiologically insoluble, particulate phosphate carriers usefull in this context include the well known compounds of the general $MO-P_2O_5-H_2O$ system, where M is a divalent cation such as Ca(II), Mg(II), Ba(II), and Sr(II). Calcium is especially preferred in this contest. These materials include the polyphosphates and apatites. Specific examples include $Ca_3(PO_4)_2$; $Ca_4H(PO_4)_3$; $Ca(H_2PO_4)_2$ and its dihydrate; $Ca_2P_2O_7$; $Ca_4P_2O_9$; $Ca_8H_2(PO_4)_6.5H_2O$; $CaHPO_4$ and its insoluble hydrates; insoluble calcium polyphosphate; calcium hydroxyapatite $Ca_{10}(PO_4)_6(OH)_2$; calcium fluoroapatite $Ca_{10}(PO_4)_6(F)_2$; and the corresponding Mg, Ba, and Sr phosphates.

Other specific particulates that are shown to bind phosphonates resulting in adducts that alter proton relaxation times in vitro include aluminium hydroxide, aluminium oxide, magnesium hydroxide and magnesium trisilicate and these are also claimed.

The metal complexes are prepared, by mixing an aqueous solution of the chosen di- or polyphosphonate with an aqueous solution of a salt of the chosen transition metal or lanthanide, or a di- or polyphosphonate may be added to an aqueous suspension of the transition metal carbonate or lanthanide oxide. The precise structures of the complexes are not known with certainty, and may vary depending upon the nature of the reagents.

The adducts may be formed by adding required amounts of metal complex, in solution, to the insoluble carrier. The adduct formation takes place rapidly without requirement for heating the solution.

The exact structure of the metal complex-particulate adduct is not known with certainty and will vary with the ratio of metal complex to particulate used in the preparation

In order to investigate the GI tract by NMR scanning, it may be necessary to administer, either orally or rectally, an aqueous suspension of the complex from 0.5 to 250 mmol. $1^{-1}$ , preferably 1 to 50 mmol. $^{-1}$ , with respect to the paramagnetic metal ion. The volume for use in a human subject would typically be 1 to 100ml.

The following Examples further illustrate the present invention.


EXAMPLE 1, SYNTHESIS OF PHOSPHONATES

The phosphonates used in this study were synthesised by a direct Mannich type reaction using an amine (primary or secondary), formaldehyde and phosphorous acid. The basic reaction is given in the paper by Moedritzer (JCS 1966 vol 31 p 1603) and a fuller account is given in European Patent Application No. 0210043A.

As a specific example we give the synthesis of para-xylylidenediaminetetramethylenephosphonate.

41 g $H_3PO_4$ were dissolved in 100ml of $H_2O$ / concentrated HCl (50:50 v.v) and 17g p-xylylidenediamine were added. The solution was brought to reflux and 80 ml formaldehyde solution 37% w/v were added over 1 hour. The solution was refluxed for a further hour during which precipitation of the product occurred. The solution was allowed to cool and the product filtered and washed with water, ethanol and ether, and dried at 100°C

Yield          45.8g

m.p.          249-251°C

calculated for $C_{12} H_{26} N_2 O_{12} P_4$          C28.12 H4.69 N5.47 P24.21

Found          C28.09 H4.79 N5.49 P24.04

## LIGANDS SYNTHESISED

Amino ethanol dimethylene phosphonate          - AEDMP

Glycine dimethylene phosphonate          - GDMP

Gamma amino butyric acid dimethylene phosphonate          -GABADMP

6-amino hexanoic acid dimethylene phosphonate          - AHADMP

Meta-xylylidene diamine tetramethylene phosphonate          - MXDTMP

Para-xylylidene diamine tetramethylene phosphonate          - PXDTMP

1,3-diamino-2-hydroxy-propane tetramethylene phosphonate          - DHPTMP

3,3 - diaminopropyl-N-methyl dipropylamine tetramethylene phosphonate          -DPNDPTMD

4,9-Dioxa-1,12-dodecane-di-amine tetramethylene phosphonate          - DDDATMP

Di-propylene triamine pentamethylene phosphonate          - DPTPMP

Triethylene tetramine hexamethylene phosphonate          - TTHMP

N,N - bis (3 amino propyl) ethylene diamine hexamethylene phosphonate          -BAPEDHMP

Ethylene diamine tetramethylene phosphonate- EDTMP

Butane diamine tetramethylene phosphonate          - BDTMP

Hexane diamine tetramethylene phosphonate          - HDTMP

Octane diamine tetramethylene phosphonate          - ODTMP

## EXAMPLE 2. PREPARATION OF METAL COMPLEXES

(a) Preparation of the metal polyaminophosphonates

The preparation of copper, nickel, manganese and cobalt complexes was achieved by heating the ligands, in suspension, with the metal carbonate.

As a specific example we give the preparation of manganese metaxylylidenediaminetetramethylene phosphonate.

2.6g MXDTMP and 0.56g of $MnCO_3$ were heated together in 200ml $H_2O$ for 1 hour. The solvent was then removed under reduced pressure to produce a free flowing white powder.

Yield 2.56g 96%

Calculated for $C_{12} H_{28} N_2 O_{12} P_4 Mn$          C23.08 H4.29 N4.62 P20.49

Found          C23.08 H4.13 N4.72 P19.51

Complexes with metals such as chromium and gadolinium were synthesised by mixing equimolar quantities of ligand and metal salt. The methods differ in exact technique.

$Gd(NO_3)_3$. $5H_2O$ is added slowly to a solution of the sodium salt of the ligand at pH 7.0. There is precipitation but this is redissolved upon adding NaOH (dilute). Ligands containing less than four aminophosphonates were added at a concentration twice that of the metal. With four or more $-CH_2PO_3H_2$ groups the metal ligand ratio is 1:1.

Chromium complexes were made by boiling equimolar amounts of ligand and $Cr(NO_3)_3$. $9H_2O$ or in some cases ligand present in 2:1 excess as above. The reaction was seen to occur by noting the colour change blue to green. The mixtures were refluxed for 1-2 hours. The pale green precipitate was redissolved by addition of dilute NaOH to give a pH of $\simeq$ 7.0.

(b) The preparation of Gadolinium Phytate

The gadolinium phytate used in this work was prepared in the same manner as the gadolinium phosphonates described previously. Sodium phytate (34.28g, 0.04mol) was dissolved in 50ml distilled water. Gadolinium nitrate, $Gd(NO_3)_3.5H_2O$, (4.33g, 0.01mol) was added and the resulting precipitate redissolved by restoring the pH to 7.5 by the addition of 0.1M NaOH. The solution was then made up to 100ml in a volumetric flask to give a stock solution which was 0.1M with respect to $Gd^{3+}$.

(c) Other complexes

The iron complexes used in the preparation of particulates were obtained from standard laboratory suppliers. This enabled the binding of ferric citrate and ferric pyrophosphate to various particulates to be studied.

RELAXATION DATA 1.

Relaxation measurements have been made on the above complexes at 200MHz and 10MHz, a clinically relevant field strength. Data quoted was obtained for metal complexes at 1 milimolar concentration in all cases.

| (a) Chromium complexes 1 milimolar concentration | | |
|---|---|---|
| | T1 200MHz 25°C | T1 10MHz 37°C |
| Water standard | 3.30s | 3.40s |
| Cr (AEDMP)$_2$ | 1.3s | 1.26s |
| Cr (NTMP)$_2$ | 0.55s | 0.79s |
| Cr (GDMP)$_2$ | 0.89s | 0.95s |
| Cr (IDAMP)$_2$ | 0.83s | 0.92s |
| Cr (GABADMP)$_2$ | 0.91s | 1.00s |
| Cr (AHADMP)$_2$ | 1.04s | 0.96s |
| Cr MXDTMP | 0.55s | 0.62s |

| (b) Gadolinium complexes 1 milimolar concentration | | |
|---|---|---|
| | T1 200MHz 25°C | T1 10MHz 37°C |
| Water standard | 3.30s | 3.40s |
| Gd (NTMP)$_2$ | 0.06s | 0.09s |
| Gd EDTMP | 0.08s | 0.09s |
| Gd DPTMP | 0.10s | 0.10s |
| Gd (GDMP)$_2$ | 0.02s | 0.12s |
| Gd (IDAMP)$_2$ | 0.02s | 0.16s |
| Gd MXDTMP | 0.06s | 0.07s |
| Gd (AHADMP)$_2$ | 0.06s | 0.12s |
| Gd (Phytate) | 0.05s | 0.07s |

EP 0 275 215 B1

| (c) Manganese complexes 1 milimolar concentration | | |
|---|---|---|
| | T1 200MHz 25°C | T1 10MHz 37°C |
| Water standard | 3.30s | 3.40s |
| Mn (IDAMP)$_2$ | 0.07s | 0.32s |
| Mn (AEDMP)$_2$ | 0.07s | 0.12s |
| Mn (GDMP)$_2$ | 0.16s | 0.18s |
| Mn (GABADMP)$_2$ | 0.18s | 0.27s |
| Mn (AHADMP)$_2$ | 0.12s | 0.13s |
| Mn EDTMP | 0.23s | 0.24s |
| Mn MXDTMP | 0.09s | 0.10s |
| Mn DPTMP | 0.29s | 0.32s |

| (d) Other complexes 1 milimolar concentration | | |
|---|---|---|
| | T1 200MHz 25°C | T1 10MHz 37°C |
| Water standard | 3.30s | 3.40s |
| Fe(III) citrate | 1.30s | 1.56s |
| Fe(III) pyrophosphate | 2.20s | 2.33s |

EXAMPLE 3. CHARACTERISATION OF PARTICULATES

The carriers employed in the current invention were investigated to determine various physical characteristics namely particle size, sedimentation rate and acid/base behaviour.

Particle size

The particle size of the particulates was measured by laser scattering using a Malvern Zetasizer IIc. The data obtained are presented in the table below.

| PARTICLE SIZE ANALYSIS OF PARTICULATE CARRIERS | |
|---|---|
| COMPOUND | PARTICLE SIZE |
| Al(OH)$_3$ | 2.38$\mu$m |
| AlPO$_4$ | 5.66$\mu$m |
| Ca(OH)$_2$ | 4.66$\mu$m |
| CaPO$_4$–basic | 3.87$\mu$m |
| CaPO$_4$-neutral | 4.01$\mu$m |
| CaPO$_4$-ortho | 3.26$\mu$m |
| Ca pyrophosphate | 2.33$\mu$m |
| Mg(OH)$_2$ | 1.29$\mu$m |
| Mg trisilicate | 1.54$\mu$m |
| NaPO$_4$ | 1.43$\mu$m |
| Silica | 4.66$\mu$m |

Sedimentation rate

Sedimentation rates were measured using the following method. 10ml of particulate suspension (100mg/ml) were prepared in a cylindrical glass sample tube 20mm x 75mm. The solid was suspended by vigorous shaking for 60s and then allowed to settle. A line had been drawn on the side of the tube prior to the study representing 50% of the volume. The time taken for all the solid to fall below this mark was then measured. This was repeated three times and in average obtained. These values are shown below.

6

Acid/base behaviour

The pH of the particulate suspensions (100mg/ml) was measured using a Corning pH meter and the values obtained are included in the table below.

| pH AND SEDIMENTATION STUDIES ON CARRIER PARTICULATES | | |
|---|---|---|
| COMPOUND | pH 100mg/ml | Sed. time 50% |
| $Al(OH)_3$ | 6.72 | 559s |
| $AlPO_4$ | 6.80 | 18s |
| $Ca(OH)_2$ | 9.15 | 520s |
| $CaPO_4$-basic | 6.85 | 98s |
| $CaPO_4$-neutral | 6.85 | 52s |
| $CaPO_4$-ortho | 6.82 | 308s |
| Ca pyrophosphate | 6.82 | 46s |
| $Mg(OH)_2$ | 9.20 | 312s |
| Mg trisilicate | 9.00 | 26s |
| $NaPO_4$ glass | 6.85 | 85s |
| Silica | 6.80 | 36s |

## EXAMPLE 4. BINDING OF METAL COMPLEXES TO PARTICULATE CARRIER

The particulates used were generally used as suspensions in distilled water for these studies (250mg/ml). The solid from 1ml of particulate solution was isolated by centrifugation and the supernatant removed. 0.4ml of a 50mM metal complex solution at pH = 7.5 was added and the solid resuspended. The adduct formed almost immediately. After 3 minutes the solid was isolated by centrifugation washed once by resuspension in distilled water (0.4ml) followed by recentrifugation to isolate the products. The initial supernatant was analysed by $^1$H NMR relaxation measurements at 200MHz and the amount of metal complex bound to the particulate calculated. The particulates were resuspended at 10 mg/ml and the T1 and T2 relaxation times measured at 10MHz and 37°C.

The binding of gadolinium complexes was also assayed using $^{153}$Gd labelled solutions. The particulates were used as suspensions (250mg/ml) and isolated by centrifugation. 0.4ml of a radioactively labelled gadolinium complex was then added and the solid resuspended. After three minutes the solid was filtered through a glass sinter and washed with water (≈10ml). The total activity of the filtrate was measured and expressed as a percentage of the original. The gadolinium concentration of the complex in this assay was 12.5mM and the concentration of phosphonate (where present) was 25mM. A further assay involved the addition of 25mM, and 37.5mM Gd with concomitant increase in the phosphonate concentration.

| BINDING OF METAL COMPLEXES TO ALUMINIUM HYDROXIDE | |
|---|---|
| Metal complex | % Bound |
| $GdCl_3$ | 98% |
| Gd $(NTMP)_2$ | 84% |
| Gd EDTMP | 88% |
| Gd MXDTMP | 83% |
| Gd DPTMP | 71% |
| Gd Phytate | 85% |
| $FeCl_3$ | 89% |
| Ferric citrate | 80% |

EP 0 275 215 B1

| BINDING OF METAL COMPLEXES TO ALUMINIUM PHOSPHATE | |
|---|---|
| Metal complex | % Bound |
| Gd (IDAMP)$_2$ | 58% |
| Gd (GDMP)$_2$ | 72% |
| Gd (AHADMP)$_2$ | 73% |
| Gd (NTMP)$_2$ | 69% |
| Gd EDTMP | 57% |
| Gd DPTMP | 64% |
| Gd Phytate | 42% |
| FeCl$_3$ | 78% |
| Ferric citrate | 18% |

| BINDING OF METAL COMPLEXES TO CALCIUM HYDROXIDE | |
|---|---|
| Metal complex | % Bound |
| GdCl$_3$ | 99% |
| Gd EDTMP | 89% |
| Gd MXDTMP | 83% |
| Gd DPTMP | 735 |
| Gd Phytate | 99% |
| FeCl$_3$ | 98% |
| Ferric citrate | 74% |

| BINDING OF METAL COMPLEXES TO CALCIUM PHOSPHATE BASIC | |
|---|---|
| Metal complex | % Bound |
| GdCl$_3$ | 99% |
| Gd (NTMP)$_2$ | 64% |
| Gd EDTMP | 67% |
| Gd MXDTMP | 61% |
| Gd DPTMP | 53% |
| Gd Phytate | 75% |
| FeCl$_3$ | 96% |
| Ferric citrate | 80% |

| BINDING OF METAL COMPLEXES TO CALCIUM PHOSPHATE NEUTRAL | |
|---|---|
| Metal complex | % Bound |
| GdCl$_3$ | 98% |
| Gd (NTMP)$_2$ | 67% |
| Gd EDTMP | 69% |
| Gd MXDTMP | 74% |
| Gd DPTMP | 59% |
| Gd Phytate | 69% |
| FeCl$_3$ | 92% |
| Ferric citrate | 68% |

8

| BINDING OF METAL COMPLEXES TO CALCIUM PHOSPHATE ORTHO | |
|---|---|
| Metal complex | % Bound |
| None (No T1 change) | None |
| Cr (AEDMP)$_2$ | 86% |
| Cr (GDMP)$_2$ | 80% |
| Cr (GABADMP)$_2$ | 80% |
| Cr (AHADMP)$_2$ | 88% |
| Cr MXDTMP | 90% |
| Gd MXDTMP | 92% |
| Gd (NTMP)$_2$ | 95% |
| Gd EDTMP | 92% |
| Gd DPTMP | 94% |
| Gd (AHADMP)$_2$ | 90% |
| Gd Phytate | 73% |
| Ferric citrate | 82% |

| BINDING OF METAL COMPLEXES TO CALCIUM PYROPHOSPHATE | |
|---|---|
| Metal complex | % Bound |
| GdCl$_3$ | 68% |
| Gd (NTMP)$_2$ | 55% |
| Gd EDTMP | 60% |
| Gd MXDTMP | 59% |
| Gd DPTMP | 58% |
| Gd Phytate | 58% |
| FeCl$_3$ | 50% |
| Ferric citrate | 28% |

| BINDING OF METAL COMPLEXES TO MAGNESIUM HYDROXIDE | |
|---|---|
| Metal complex | % Bound |
| GdCl$_3$ | 99% |
| Gd (NTMP)$_2$ | 62% |
| Gd EDTMP | 77% |
| Gd MXDTMP | 74% |
| Gd DPTMP | 63% |
| Gd Phytate | 64% |
| FeCl$_3$ | 96% |
| Ferric citrate | 89% |

| BINDING OF METAL COMPLEXES TO MAGNESIUM TRISILICATE | |
|---|---|
| Metal complex | % Bound |
| $GdCl_3$ | 89% |
| Gd $(NTMP)_2$ | 66% |
| Gd EDTMP | 64% |
| Gd MXDTMP | 60% |
| Gd DPTMP | 51% |
| Gd Phytate | 64% |
| $FeCl_3$ | 93% |
| Ferric citrate | 50% |

| BINDING OF METAL COMPLEXES TO SODIUM PHOSPHATE GLASS | |
|---|---|
| Meal complex | % Bound |
| $GdCl_3$ | 48% |
| Gd $(NTMP)_2$ | 29% |
| Gd EDTMP | 26% |
| Gd MXDTMP | 29% |
| Gd DPTMP | 21% |
| Gd Phytate | 34% |
| $FeCl_3$ | 41% |
| Ferric citrate | 26% |

| BINDING OF METAL COMPLEXES TO SILICA | |
|---|---|
| Meal complex | % Bound |
| $GdCl_3$ | 66% |
| Gd $(NTMP)_2$ | 43% |
| Gd EDTMP | 39% |
| Gd MXDTMP | 35% |
| Gd DPTMP | 24% |
| Gd Phytate | 60% |
| $FeCl_3$ | 46% |
| Ferric citrate | 16% |

| BINDING TO $CaPO_4$ AND CaPYROPHOSPHATE - RADIOACTIVE ASSAY | | |
|---|---|---|
| | $CaPO_4$ | $Ca_2P_2O_7$ |
| GdCl3 | 2.07% | 0.14% |
| GdEDTMP | 3.42% | 6.15% |
| GdDPTMP | 6.08% | 1.48% |
| GdMXDTMP | 0.80% | 1.20% |

| EFFECT OF INCREASING COMPLEX CONCENTRATION ON BINDING TO $CaPO_4$ | | | |
|---|---|---|---|
| | 12.5mM | 25mM | 37.5mM |
| GdEDTMP | 3.42% | 15.6% | 33.39% |
| GdDPTMP | 6.08% | 0.50% | 4.91% |

RELAXATION PROPERTIES OF THE METAL COMPLEX - PARTICULATE ADDUCTS

The $T_1$ and $T_2$ relaxation times were obtained for the adducts prepared as suspensions in distilled water (10mg/ml) at 10 MHz 37°C.

| 1. ALUMINIUM HYDROXIDE ADDUCTS RELAXATION BEHAVIOUR | | |
|---|---|---|
| Complex Bound | $T_1$ | $T_2$ |
| None | 3.00s | 0.96s |
| $Gd(NTMP)_2$ | 0.19s | 0.18s |
| Gd EDTMP | 0.21s | 0.20s |
| Gd MXDTMP | 0.22s | 0.19s |
| Gd DPTMP | 0.23s | 0.20s |
| Gd Phytate | 0.28s | 0.27s |
| $FeCl_3$ | 0.41s | 0.15s |
| Fe(citrate) | 1.10s | 0.70s |
| Fe Pyrophosphate | 0.60s | 0.50s |

| 2. ALUMINIUM PHOSPHATE ADDUCTS RELAXATION BEHAVIOUR | | |
|---|---|---|
| Complex Bound | $T_1$ | $T_2$ |
| $GdCl_3$ | 0.33s | 0.25s |
| $Gd(NTMP)_2$ | 0.71s | 0.53s |
| Gd EDTMP | 0.44s | 0.39s |
| Gd MXDTMP | 0.39s | 0.39s |
| Gd DPTMP | 0.47s | 0.43s |
| Gd Phytate | 1.70s | 1.30s |
| $FeCL_3$ | 0.89s | 0.50s |
| Fe(citrate) | 1.99s | 0.94s |
| Fe Pyrophosphate | 0.64s | 0.54s |

| 3. CALCIUM HYDROXIDE ADDUCTS RELAXATION BEHAVIOUR | | |
|---|---|---|
| Complex Bound | $T_1$ | $T_2$ |
| None | 2.55s | 1.66s |
| $GdCl_3$ | 0.22s | 0.16s |
| $Gd(NTMP)_2$ | 0.44s | 0.37s |
| Gd EDTMP | 0.33s | 0.30s |
| Gd MXDTMP | 0.34s | 0.32s |
| Gd DPTMP | 0.29s | 0.25s |
| Gd Phytate | 2.01s | 1.00s |
| $FeCl_3$ | 0.62s | 0.54s |
| Fe(citrate) | 0.77s | 0.69s |

| 4. CALCIUM PHOSPHATE BASIC ADDUCTS RELAXATION BEHAVIOUR | | |
|---|---|---|
| Complex Bound | $T_1$ | $T_2$ |
| None | 2.50s | 1.70s |
| $GdCl_3$ | 0.11s | 0.10s |
| Gd $(NTMP)_2$ | 0.21s | 0.20s |
| Gd EDTMP | 0.22s | 0.21s |
| Gd MXDTMP | 0.19s | 0.17s |
| Gd DPTMP | 0.25s | 0.22s |
| Gd Phytate | 0.21s | 0.19s |
| $FeCl_3$ | 0.45s | 0.43s |
| Fe(citrate) | 0.30s | 0.20s |
| Fe Pyrophosphate | 0.40s | 0.30s |

| 5. CALCIUM PHOSPHATE NEUTRAL ADDUCTS RELAXATION BEHAVIOUR | | |
|---|---|---|
| Complex Bound | $T_1$ | $T_2$ |
| None | 2.22s | 1.29s |
| $GdCl_3$ | 0.10s | 0.08s |
| Gd $(NTMP)_2$ | 0.15s | 0.13s |
| Gd EDTMP | 0.19s | 0.15s |
| Gd MXDTMP | 0.17s | 0.12s |
| Gd DPTMP | 0.26s | 0.20s |
| Gd Phytate | 0.41s | 0.34s |
| $FeCl_3$ | 0.32s | 0.22s |
| Fe(citrate) | 1.72s | 1.08s |
| Fe Pyrophosphate | 1.60s | 1.00s |

| 6. CALCIUM (ORTHO)PHOSPHATE ADDUCTS RELAXATION BEHAVIOUR | | |
|---|---|---|
| Complex Bound | $T_1$ | $T_2$ |
| None $Ca_3(PO_4)_2$ only | 2.50s | 0.97s |
| Cr $(AEDMP)_2$ | 1.03s | 0.53s |
| Cr $(GDMP)_2$ | 0.97s | 0.54s |
| Cr $(GABADMP)_2$ | 1.39s | 0.67s |
| Cr $(AHADMP)_2$ | 1.17s | 0.55s |
| Cr MXDTMP | 0.98s | 0.56s |
| Gd $(AEDMP)_2$ | 0.14s | 0.12s |
| Gd $(GDMP)_2$ | 0.22s | 0.19s |
| Gd $(GABADMP)_2$ | 0.18s | 0.16s |
| Gd $(AHADMP)_2$ | 0.26s | 0.18s |
| Gd $(NTMP)_2$ | 0.19s | 0.18s |
| Gd EDTMP | 0.23s | 0.13s |
| Gd DPTMP | 0.22s | 0.20s |
| Gd MXDTMP | 0.21s | 0.21s |
| Gd Phytate | 0.41s | 0.39s |
| $FeCl_3$ | 0.33s | 0.22s |
| Fe (citrate) | 0.60s | 0.30s |
| Fe Pyrophosphate | 0.40s | 0.30s |

| 7. CALCIUM PYROPHOSPHATE ADDUCTS RELAXATION BEHAVIOUR | | |
|---|---|---|
| Complex Bound | $T_1$ | $T_2$ |
| Calcium pyrophosphate only | 3.3s | 1.82s |
| $GdCl_3$ | 0.09s | 0.08s |
| Gd (NTMP)$_2$ | 0.19s | 0.18s |
| Gd (AHADMP)$_2$ | 0.14s | 0.13s |
| Gd EDTMP | 0.15s | 0.13s |
| Gd DPTMP | 0.16s | 0.12s |
| Gd MXDTMP | 0.16s | 0.13s |
| Gd Phytate | 1.66s | 1.19s |
| $FeCl_3$ | 0.53s | 0.44s |
| Fe(citrate) | 0.87s | 0.53s |
| Fe Pyrophosphate | 1.80s | 1.24s |
| $CrCl_3$ | 1.57s | 1.43s |
| Cr (NTMP)$_2$ | 2.72s | 1.40s |
| Cr (GABADMP)$_2$ | 1.33s | 1.20s |
| Cr EDTMP | 1.54s | 0.86s |
| Cr MXDTMP | 1.61s | 0.90s |
| Cr DPTMP | 1.54s | 0.87s |

| 8. MAGNESIUM HYDROXIDE ADDUCTS RELAXATION BEHAVIOUR | | |
|---|---|---|
| Complex Bound | $T_1$ | $T_2$ |
| $GdCl_3$ | 0.22s | 0.18s |
| Gd (NTMP)$_2$ | 0.34s | 0.33s |
| Gd EDTMP | 0.41s | 0.39s |
| Gd MXDTMP | 0.45s | 0.40s |
| Gd DPTMP | 0.55s | 0.41s |
| Gd Phytate | 0.70s | 0.51s |
| $FeCl_3$ | 0.40s | 0.31s |
| Fe(citrate) | 0.84s | 0.92s |
| Fe Pyrophosphate | 0.78s | 0.66s |

| 9. MAGNESIUM TRISILICATE ADDUCTS RELAXATION BEHAVIOUR | | |
|---|---|---|
| Complex bound | $T_1$ | $T_2$ |
| None | 1.20s | 0.35s |
| $GdCl_3$ | 0.09s | 0.08s |
| Gd (NTMP)$_2$ | 0.31s | 0.26s |
| Gd EDTMP | 0.29s | 0.24s |
| Gd MXDTMP | 0.29s | 0.20s |
| Gd DPTMP | 0.06s | 0.05s |
| Gd Phytate | 0.20s | 0.16s |
| $FeCl_3$ | 0.16s | 0.05s |
| Fe(citrate) | 1.06s | 0.46s |
| Fe Pyrophosphate | 0.54s | 0.44s |

| 10. SODIUM PHOSPHATE GLASS ADDUCTS RELAXATION BEHAVIOUR | | |
|---|---|---|
| Complex Bound | $T_1$ | $T_2$ |
| $GdCl_3$ | 0.36s | 0.32s |
| Gd $(NTMP)_2$ | 0.47s | 0.46s |
| Gd EDTMP | 0.46s | 0.45s |
| Gd MXDTMP | 0.44s | 0.42s |
| Gd DPTMP | 0.54s | 0.48s |
| Gd Phytate | 1.67s | 1.01s |
| $FeCl_3$ | 0.32s | 0.22s |
| Fe(citrate) | 1.57s | 1.32s |
| Fe Pyrophosphate | 1.45s | 1.44s |

| 11. SILICA ADDUCTS RELAXATION BEHAVIOUR | | |
|---|---|---|
| Complex Bound | $T_1$ | $T_2$ |
| None | 3.22s | 1.87s |
| $GdCl_3$ | 0.19s | 0.18s |
| Gd $(NTMP)_2$ | 0.36s | 0.32s |
| Gd EDTMP | 0.34s | 0.30s |
| Gd MXDTMP | 0.33s | 0.29s |
| Gd DPTMP | 0.46s | 0.41s |
| Gd Phytate | 0.21s | 0.15s |
| $FeCl_3$ | 0.72s | 0.70s |
| Fe(citrate) | 0.34s | 0.32s |

The results show that the complexes once bound to the insoluble particulate still give efficient relaxation of water molecules. The reduction is, in many cases, very significant and indicates the utility of these compounds in vivo. After oral or rectal administration these compounds are likely to permit visualisation of the G.I. tract by NMR scanning. For this purpose the adducts compare favourably with those described in EPA 183760.

The stability of the particulate carriers employed in this invention is of great importance. Gastric contents are reported to contain varying amounts of HCl dependant upon various factors. The stability of calcium phosphate and calcium pyrophosphate was investigated using various HCl concentrations. 0.5g of particulate was taken and 5ml of HCl solution added. The particulate was suspended by stirring and left for 24 hours before the supernatant was removed by centrifugation. Dissolution was measured using $^{31}P$ NMR at 24.15MHz on a JEOL FX-60 spectrometer. The following table shows the results obtained. All values are expressed as percentages with the value obtained using concentrated HCl being used as the 100% value.

| DISSOLUTION OF CALCIUM PHOSPHATE AND CALCIUM PYROPHOSPHATE IN ACID SOLUTION | | |
|---|---|---|
| HCl CONCENTRATION | $CaPO_4$ IN SOLUTION | $Ca_2P_2O_7$ IN SOLUTION |
| 0.0M | 0.0% | 0.0% |
| 0.2M | 4.7% | 7.8% |
| 0.4M | 19.2% | 12.2% |
| 0.6M | 46.7% | 16.7% |
| 0.8M | 90.8% | 23.0% |
| 1.0M | 94.7% | 27.2% |
| 2.0M | 100.0% | 37.4% |
| CONC. $\simeq$10M | 100.0% | 100.0% |

STABILITY OF GADOLINIUM PHOSPHONATE-PARTICULATE ADDUCTS IN ACID SOLUTION

The stability of the adducts in acid solution was assayed using $^{153}$Gd labelled adducts. The adducts were prepared as in the binding assays described previously. 5ml of 0.2M HCl was then added to the adduct which was resuspended and left for 24hrs. The suspensions were then filtered through a glass sinter and washed with 5ml distilled water. The total filtrate was then placed into an ion chamber and the total activity measured. The following table shows the results obtained expressed as a percentage of the original activity.

| | PERCENTAGE ACTIVITY IN SUPERNATANT | |
|---|---|---|
| | $Ca_2(PO_4)_3$ | $Ca_2P_2O_7$ |
| $GdCl_3$ | 0.54% | 0.41% |
| GdEDTMP | 2.02% | 2.30% |
| GdDPTMP | 1.97% | 1.54% |

The clearance of $^{153}$Gd labelled calcium phosphate after oral dosing has been investigated in the rat. Calcium phosphate adducts were prepared using 25mM Gd solutions spiked with radioactive gadolinium. The dosage was 1 ml of suspensions or solution by catheter. The following table shows biodistribution data obtained from rats that were sacrificed 24 hours post dosing. Percentage activity per organ is given in all cases and each value represents the mean of three experiments.

| Organ | $^{153}GdCl_3$ SOLUTION | $^{153}GdMXDTMP$ SOLUTION | $^{153}GdCl_3$ + $Ca_3PO_4$ | $^{153}GdMXDTMP$ + $Ca_3PO_4$ |
|---|---|---|---|---|
| Lung 0 | 0 | 0 | 0 | |
| Liver/spleen | 0 | 0 | 0 | 0 |
| Stomach | 1.4 | 0.1 | 0 | 0 |
| Small intestine | 44.1 | 0 | 0.1 | 0 |
| Large intestine | 0 | 0 | 9.4 | 2.8 |
| Kidneys | 0 | 0 | 0 | 0 |
| Bladder & urine | 0 | 0 | 0 | 0 |
| Bone 0 | 0 | 0 | 0 | |
| Muscle | 0 | 0 | 0 | 0 |
| Blood | 0 | 0 | 0 | 0 |
| Carcass | 0 | 0 | 0 | 0 |
| Caecum | 0 | 1.8 | 0 | 0 |
| Rectum | 0 | 2.9 | 0 | 0 |
| Faeces | 54.5% | 95.2% | 90.5% | 97.2% |

These figures show good clearance with no or minimal absorption from the gut. The use of calcium phosphate appears to reduce the retention time of bath complexed and uncomplexed $^{153}$Gd which would be advantageous in human studies.

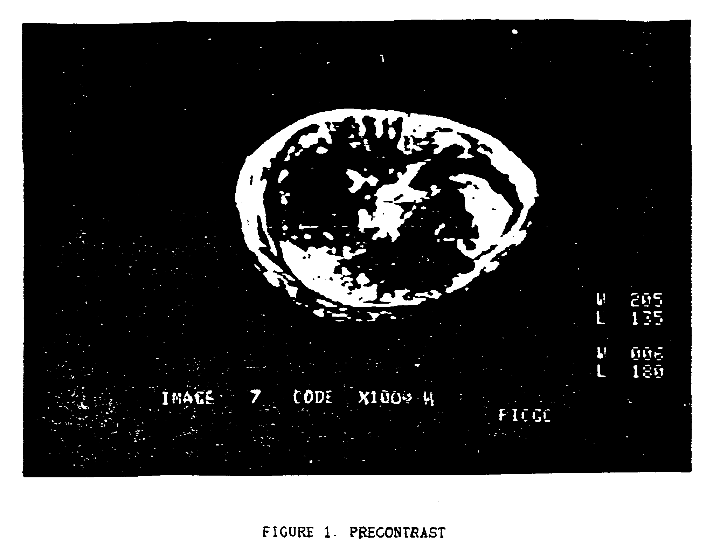

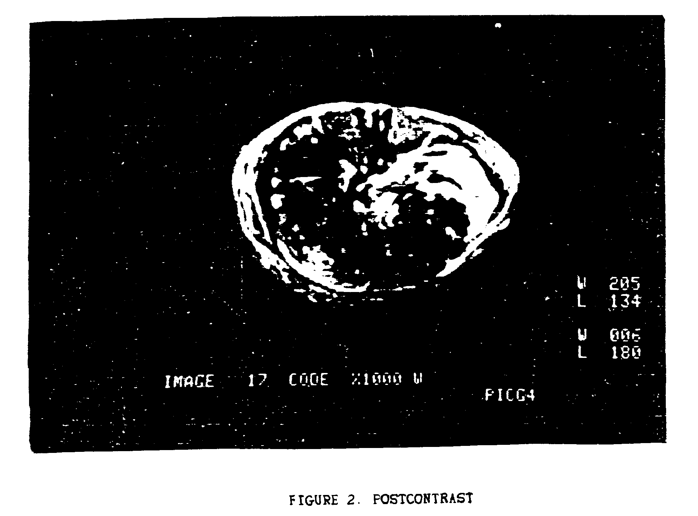

The final data to be presented concerns the use of these compounds as a contrast agent in the rabbit. An adduct was prepared in the manner described previously using GdMXDTMP and calcium phosphate. A New Zealand white rabbit ≈5kg was anaesthetised and a catheter inserted into the stomach. The animal was then placed into an NMR imaging machine operating at .015 Tesla. A precontrast picture was obtained (Fig.1) using a saturation recovery pulse sequence which showed the stomach as a dark area with no fluid discrimination. 30ml of the particulate suspension (4mg/ml) was then administered via the catheter and a post contrast picture obtained, Fig.2. This shows the fluid level in the stomach most clearly. Father pictures showed fluid levels believed to be from loops of bowel.

Thus the data given above shows that phosphonate-particulate adducts suitable for NMR imaging of the gastrointestinal tract can be simply prepared, have good stability in vitro, are excreted within 24hrs in the rat and give contrast in vivo.

**Claims**

1.  A contrast agent for investigating the gastrointestinal tract by NMR scanning which comprises a complex formed between a) an amino di- or polyphosphonate in which the phosphonate groups comprise several carbon atoms, and b) a paramagnetic metal ion, in substantially permanent association with a particulate carrier insoluble in an aqueous medium, provided that said carrier is other than hydroxyapatite.

2. A contrast agent as claimed in claim 1, wherein the di- or polyphosphonate contains the group $-N-(CH_2PO_3H_2)_2$.

3. A contrast agent as claimed in claim 1 or claim 2, wherein the polyphosphonate has the general formula $NX_2CH_2PO_3H_2$, where the two groups X are the same or different and each is $-CH_2PO_3H_2$ or $-(CH_2)_n N(CH_2PO_3H_2)_2$, and n is an integer from 1 to 10, or a non-toxic salt thereof.

4. A contrast agent as claimed in claim 3, wherein the polyphosphonate is ethylenediamine tetramethylenephosphonate.

5. A contrast agent as claimed in claim 1 or claim 2, wherein the polyphosphonate is metaxylenediamine tetramethylenephosphonate.

6. A contrast agent as claimed in any of claims 1 to 5, wherein the paramagnetic metal ion is Gd(III) or Fe(III).

7. A contrast agent as claimed in any of claims 1 to 6, wherein the particulate carrier insoluble in an aqueous medium is a pharmaceutically acceptable, substantially physiologically insoluble, particulate phosphate carrier.

8. A contrast agent as claimed in claim 7, wherein said carrier is of the $M-P_2O_5-H_2O$ type, where M is a divalent cation.

9. A contrast agent as claimed in claim 8, wherein M is calcium (II).

10. A contrast agent as claimed in any of claims 1 to 6, wherein the particulate carrier insoluble in an aqueous medium is aluminium hydroxide, aluminium oxide, magnesium hydroxide or magnesium trisilicate.

11. A contrast agent as claimed in any of the preceding claims, wherein the complex is present in solution at a concentration of from 1 to 50 mM with respect to the paramagnetic metal ion.

12. A contrast agent as claimed in claim 8, wherein said carrier is selected from $Ca_3(PO_4)_2$; $Ca_4H(PO_4)_3$; $Ca(H_2PO_4)_2$ and its dihydrate; $Ca_2P_2O_7$; $Ca_4P_2O_9$; $Ca_8H_2(PO_4)_6 \cdot 5H_2O$; $CaHPO_4$ and its insoluble hydrates; insoluble calcium polyphosphate; calcium fluoroapatite $Ca_{10}(PO_4)_6F_2$; and the corresponding Mg, Ba and Sr phosphates.

**Revendications**

1. Agent de contraste pour l'exploration du tractus gastro-intestinal par balayage par RMN, comprenant un complexe formé entre a) un amino-di- ou polyphosphonate dans lequel les groupes phosphonate comprennent plusieurs atomes de carbone, et b) un ion métallique paramagnétique, en association pratiquement permanente avec un véhicule particulaire insoluble dans un milieu aqueux, étant entendu que ledit véhicule est autre que l'hydroxyapatite.

2. Agent de contraste selon la revendication 1, dans lequel le di- ou polyphosphonate contient le groupe $-N(CH_2PO_3H_2)_2$.

3. Agent de contraste selon la revendication 1 ou 2, dans lequel le polyphosphonate correspond à la formule générale $NX_2CH_2PO_3H_2$, dans laquelle les deux groupes X sont identiques ou différents et chacun est $-CH_2PO_3H_2$ ou $-(CH_2)_nN(CH_2PO_3H_2)_2$, et n est un nombre entier allant de 1 à 10, ou est un sel non toxique de celui-ci.

4. Agent de contraste selon la revendication 3, dans lequel le polyphosphonate est l'éthylènediamine-

EP 0 275 215 B1

tétraméthylènephosphonate.

**5.** Agent de contraste selon la revendication 1 ou 2, dans lequel le polyphosphonate est le métaxylènediamine-tétraméthylènephosphonate.

**6.** Agent de contraste selon l'une quelconque des revendications 1 à 5, dans lequel l'ion métallique paramagnétique est Gd-(III) ou Fe-(III).

**7.** Agent de contraste selon l'une quelconque des revendications 1 à 6, dans lequel le véhicule particulaire insoluble dans un milieu aqueux est un véhicule de type phosphate particulaire pharmaceutiquement acceptable, pratiquement insoluble dans les conditions physiologiques.

**8.** Agent de contraste selon la revendication 7, dans lequel ledit véhicule est du type $M-P_2O_5-H_2O$, M étant un cation divalent.

**9.** Agent de contraste selon la revendication 8, dans lequel M est le calcium-(II).

**10.** Agent de contraste selon l'une quelconque des revendications 1 à 6, dans lequel le véhicule particulaire insoluble dans un milieu aqueux est l'hydroxyde d'aluminium, l'oxyde d'aluminium, l'hydroxyde de magnésium ou le trisilicate de magnésium.

**11.** Agent de contraste selon l'une quelconque des revendications précédentes, dans lequel le complexe est présent en solution à une concentration allant de 1 à 50 mM exprimée en ion métallique paramagnétique.

**12.** Agent de contraste selon la revendication 8, dans lequel ledit véhicule est choisi parmi $Ca_3(PO_4)_2$, $Ca_4H(PO_4)_3$, $Ca(H_2PO_4)_2$ et son dihydrate, $Ca_2P_2O_7$, $Ca_4P_2O_9$, $Ca_8H_2(PO_4)_6.5H_2O$, $CaHPO_4$ et ses hydrates insolubles, le polyphosphate de calcium insoluble, la fluoroapatite de calcium $Ca_{10}(PO_4)_6F_2$, et les phosphates de Mg, Ba et Sr correspondants.

**Patentansprüche**

**1.** Kontrastmittel zur Untersuchung des gastrointestinalen Trakts durch magnetische Kernresonanzabtastung, welches einen zwischen a) einem Aminodi- oder -polyphosphonat, in welchem die Phosphonatgruppen mehrere Kohlenstoffatome umfassen, und b) einem paramagnetischen Metallion ausgebildeten Komplex in im wesentlichen permanenter Assoziation mit einem aus Partikeln bestehenden Träger, welcher in einem wässerigen Medium unlöslich ist, umfaßt, vorausgesetzt, daß der Träger ein anderer als Hydroxyapatit ist.

**2.** Kontrastmittel nach Anspruch 1, bei welchem das Di- oder Polyphosphonat die Gruppe $-N(CH_2PO_3H_2)_2$ enthält.

**3.** Kontrastmittel nach Anspruch 1 oder 2, bei welchem das Polyphosphonat die allgemeine Formel $NX_2CH_2PO_3H_2$ hat, wobei die beiden X-Gruppen gleich oder verschieden sind und jede $-CH_2PO_3H_2$ oder $(CH_2)_n N(CH_2PO_3H_2)_2$, wobei n eine ganze Zahl von 1 bis 10 ist, oder ein nicht-toxisches Salz derselben sind.

**4.** Kontrastmittel nach Anspruch 3, bei welchem das Polyphosphonat Ethylendiamintetramethylenphosphonat ist.

**5.** Kontrastmittel nach Anspruch 1 oder 2, bei welchem das Polyphosphonat Metaxylendiamintetramethylenphosphonat ist.

**6.** Kontrastmittel nach irgendeinem der Ansprüche 1 bis 5, bei welchem das paramagnetische Metallion Gd(III) oder Fe(III) ist.

**7.** Kontrastmittel nach irgendeinem der Ansprüche 1 bis 6, bei welchem der in einem wässerigen Medium unlösliche aus Partikeln bestehende Träger ein pharmazeutisch annehmbarer, im wesentlichen physio-

17

logisch unlöslicher aus Partikeln bestehender Phosphatträger ist.

8. Kontrastmittel nach Anspruch 7, bei welchem der Träger vom M-$P_2O_5$-$H_2O$-Typ ist, wobei M ein zweiwertiges Kation ist.

9. Kontrastmittel nach Anspruch 8, bei welchem M Kalzium ist.

10. Kontrastmittel nach irgendeinem der Ansprüche 1 bis 6, bei welchem der in einem wässerigen Medium unlösliche aus Partikeln bestehende Träger Aluminiumhydroxid, Aluminiumoxid, Magnesiumhydroxid oder Magnesiumtrisilicat ist.

11. Kontrastmittel nach irgendeinem der vorstehenden Ansprüche, wobei der Komplex in Lösung bei einer Konzentration von 1 bis 50 mM in Bezug auf das paramagnetische Metallion zugegen ist.

12. Kontrastmittel nach Anspruch 8, bei welchem der Träger aus $Ca_3(PO_4)_2$; $Ca_4H(PO_4)_3$; $Ca(H_2PO_4)_2$ und ihren Dihydraten; $Ca_2P_2O_7$; $Ca_4P_2O_9$; $Ca_8H_2(PO_4)_6 \cdot 5H_2O$; $CaHPO_4$ und ihren unlöslichen Hydraten; unlöslichem Kalziumpolyphosphat; Kalziumfluorapatit $Ca_{10}(PO_4)_6F_2$; und den entsprechenden Mg-, Ba- und Sr-Phosphaten ausgewählt ist.

FIGURE 1. PRECONTRAST

FIGURE 2. POSTCONTRAST